# EUROPEAN PATENT APPLICATION

(11) **EP 4 616 958 A1**
(43) Date of publication of application: **17.09.2025**
(21) Application number: 25154607.3
(22) Date of filing: 29.01.2025
(51) Int. Cl.: B05B 7/00, A61L 2/20, B05B 7/16, B05B 12/00, B05B 12/06, B05B 12/10, B65B 55/10, C12M 1/12

(54) **APPARATUS AND METHOD FOR DISPENSING A FLOW OF A SUBSTANCE**

(30) Priority: 14.03.2024 IT 202400005776
(71) Applicant: Wab Societa' A Responsabilita' Limitata, 43029 Traversetolo (Parma) (IT)
(72) Inventor: DONDI, Michele, 43030 BORE (PARMA) (IT); SALVI, Vittorio, 42026 CIANO D'ENZA (REGGIO NELL'EMILIA) (IT)
(74) Representative: Bacchini, Davide

(57) **Abstract**

Apparatus (1) for dispensing a flow of a substance, comprising:
- at least one dispensing nozzle (2);
- a conduit (3) communicating downstream with the dispensing nozzle (2);
- an atomising nozzle (5) for a liquid, arranged along the conduit (3) to pulse inject the atomised liquid into the conduit (3) to generate said substance;
a control system (6) for an occurred state transition to vapour of the atomised liquid, said control system (6) comprising detection means (7) for detecting a temperature variation at or downstream of the atomising nozzle (5).

## Description

The present invention relates to an apparatus and a method for dispensing a flow of a substance. The present invention can be applied in situations in which the atomised liquid is injected into a gaseous flow. A particular application is the dispensing of vaporised hydrogen peroxide or VHP for the purposes of treatment of containers, in particular decontamination.

As is known, containers are decontaminated before being filled. The use of vaporised hydrogen peroxide for purposes of decontamination is also known. In particular, vaporised hydrogen peroxide is obtained by introducing atomised liquid hydrogen peroxide into a flow of hot air, resulting in its vaporisation.

In this context, it can happen that the process of producing vaporised hydrogen peroxide fails and the state transition does not occur. Several known solutions provide for the possibility of performing sample monitoring of this process. These solutions are not lacking in drawbacks. In fact, they do not allow control in proximity to dispensing. Furthermore, sample checking does not ensure the total absence of errors.

In this context, the technical task underpinning the present invention is to provide an apparatus and a method for dispensing a flow of a substance which allow the limits of the prior art to be overcome.

In particular, an object of the present invention is to control that state transition has occurred in proximity to dispensing.

Another object of the present invention is to make available an apparatus and a method for dispensing a flow of a substance which ascertain that the container has been decontaminated correctly.

The specified technical task and the specified aims are substantially achieved by an apparatus and a method for dispensing a flow of a substance comprising the technical features set forth in one or more of the appended claims.

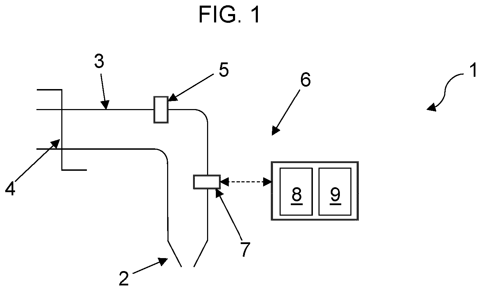

Additional features and advantages of the present invention will emerge more clearly from the approximate, and thus non-limiting, description of a preferred but not exclusive embodiment of an apparatus and a method for dispensing a flow of a substance as illustrated in the appended figure 1, in which a dispensing apparatus according to the present invention is schematically illustrated.

With reference to the figures, the number 1 indicates an apparatus for dispensing a flow of a substance.

The apparatus 1 comprises at least one substance dispensing nozzle 2. In particular, the dispensing nozzle 2 constitutes the terminal of the apparatus 1.

The apparatus 1 comprises a conduit 3. The conduit 3 is situated upstream of the dispensing nozzle 2. The conduit 3 is in direct or indirect fluid communication with the dispensing nozzle 2. In other words, the conduit 3 carries the content that flows along it towards the dispensing nozzle 2.

A gaseous fluid that forms part of the final substance to be dispensed is intended to flow along the conduit 3.

Preferably, the gaseous fluid is air. Preferably, the gaseous fluid is hot. For the specific application of generation of vaporised hydrogen peroxide, hot is intended here as at an average temperature comprised between 70 °C and 120 °C.

The apparatus 1 can comprise a tank (not shown) for the gaseous fluid, situated upstream of the conduit 3.

Preferably, the apparatus 1 comprises heating means 4 for heating the gaseous flow. In particular, the heating means 4 is positioned at the tank or along the conduit 3 or even in an intermediate pipe between the tank and the conduit 3.

The apparatus 1 comprises an atomising nozzle 5 for a liquid. The atomising nozzle 5 is arranged along the conduit 3 to pulse inject the atomised liquid into the conduit 3 (and therefore, into the gaseous flow).

The atomised liquid injected into the conduit 3 is hit by the hot gaseous fluid and consequently vaporises.

In particular, it is important for there to be a difference in temperature between the gaseous flow in the conduit 3 and the liquid to be injected. Preferably, the difference in temperature between gaseous fluid and liquid to be injected is at least 10 °C.

The apparatus 1 comprises a control system 6 for an occurred state transition to vapour of the injected liquid. The control system 6 comprises detection means 7 for detecting a temperature variation in the conduit 3 at or downstream of the atomising nozzle 5.

The state transition from liquid to vapour causes a temperature variation. The presence of the detection means 6 allows confirming, following the pulse injection of atomised liquid into the conduit 3, of whether the state transition (identified by the detection of a significant temperature variation, i.e. higher than a pre-established value) has occurred correctly.

In the preferred embodiment, the detection means 7 comprises a temperature sensor (indicated in the figures and in the text below with the same reference number 7) active on the conduit 3.

Preferably, the temperature sensor 7 is configured for continuous functioning. This means that the temperature in the conduit 3 is continuously detected, independently of pulse injection of the atomised liquid.

Alternatively, the temperature sensor 7 is configured to detect the temperature intermittently, in particular for a predefined period of time at least in part subsequent to a pulse injection of the atomised liquid.

In the preferred embodiment, the temperature sensor 7 comprises at least one filament of material having conductivity varying with the temperature.

In accordance with a first embodiment, illustrated in the figures, the detection means 7 is arranged along the conduit 3 at a first distance from the atomising nozzle 5. In particular, the detection means 7 is arranged along the conduit 3 downstream of the atomising nozzle 5.

In accordance with a second embodiment, the atomising nozzle 5 comprises the detection means 7. In other words, the detection means 7 (in particular the temperature sensor) is integrated into the atomising nozzle 5.

Preferably, the control system 6 can comprise comparison means 8 for comparing at least a part of the data acquired by the detection means 7 with a range of temperature variation values. Preferably, the control system 6 can comprise reporting means 9 for reporting an error adapted to activate when no value acquired is outside a range of temperature variation values set (i.e., when the state transition has not occurred correctly).

In one embodiment, the comparison means 8 is configured to send a first signal when at least one value acquired is higher than the given range of values. In this case, the first signal is an identifier of the occurred state transition. In fact, the detection of a temperature variation value outside the range set confirms that a significant temperature variation has occurred, i.e. due to the state transition and not linked to standard fluctuations. In particular, a detection of a temperature variation value higher than the range set occurs.

Preferably, the comparison means 8 is configured to send a second signal when no value acquired within a given time interval following a pulse injection is outside the range set. This situation identifies a lack of state transition, i.e. a non-compliant situation.

From experimental tests, it was possible to observe that injection of the atomised liquid into the gaseous flow leads first to a lowering of the temperature of the gaseous flow (due to injection of the liquid at a lower temperature), then to a temperature increase due to the state transition. The temperature variation simultaneous with the state transition stands at different values with respect to the one linked to injection of the liquid, so by selecting an opportune predefined range, it is possible to discriminate between the two temperature variations. In particular, from the tests performed, the temperature variation simultaneous with the state transition stands at around 10 °C. However, the values of these temperature variations depend on the starting temperature of the gaseous flow and the liquid that is injected.

In the preferred embodiment, the apparatus 1 is an apparatus for treating containers (in particular, for decontamination), in which the control system 6 described is implemented. In this case, the liquid is a sterilising agent and the flow exiting from the conduit 3 is used for the decontamination of containers through the dispensing nozzle 2.

In particular, the liquid is hydrogen peroxide. In this case, the process described above is functional to the generation of vaporised hydrogen peroxide or VHP. The control system 6 therefore serves to monitor a correct generation of VHP.

An object of the present invention is also a method for dispensing a flow of a substance. Such method is advantageously implemented by a dispensing apparatus of a flow of a substance as described above.

The method comprises a step of letting a flow of gaseous fluid flow along a conduit 3. The conduit 3 is in direct or indirect fluid communication downstream with at least one dispensing nozzle 2.

Preferably, the gaseous fluid is air. Preferably, the gaseous fluid is hot. For the specific application of generation of vaporised hydrogen peroxide, hot is intended here as at an average temperature comprised between 70 °C and 120 °C.

The method comprises a step of pulse injecting an atomised liquid into the flow of gaseous fluid. Such step causes a state transition to vapour and the generation of the substance to be dispensed.

In particular, it is important for there to be a difference in temperature between the gaseous flow in the conduit 3 and the liquid to be injected. Preferably, the difference in temperature between gaseous fluid and liquid to be injected is at least 10 °C.

The method comprises, following at least one pulse injection, a step of controlling an occurred state transition to vapour of the injected liquid. The controlling step comprises a step of detecting a temperature variation in the conduit 3.

This means that, in the case of detection of a temperature variation outside a given range of values, there is certainty that the state transition has occurred. In fact, the atomised liquid injected is hit by the gaseous fluid and consequently vaporises. The state transition from liquid to vapour causes a temperature variation. Detection of the temperature variation confirms that the state transition has occurred at the pulse injection of atomised liquid.

Preferably, the controlling step comprises a step of comparing at least a part of the data acquired with a range of temperature variation values.

If no value acquired is outside the given range of values, the controlling step comprises a step of reporting the presence of an error.

In one embodiment, the controlling step can comprise sending a first signal when the state transition occurs correctly following at least one pulse injection (i.e. when at least one temperature variation value acquired is outside said range of values) and a second signal when the state transition does not occur correctly (i.e. when no value acquired is outside the range of values).

In the preferred embodiment, the liquid is a sterilising agent and the flow exiting from the conduit 3 is used for the sterilisation of containers through the dispensing nozzle 2.

In particular, the liquid is hydrogen peroxide. In this case, the process described above is functional to the generation of vaporised hydrogen peroxide or VHP. In that case, the method comprises a step of generating vaporised hydrogen peroxide along the conduit 3. Such generation involves the step of letting the flow of gaseous fluid flow along the conduit 3 and the step of pulse injecting the atomised liquid. Preferably, the method comprises a step of sterilising one or more containers by dispensing vaporised hydrogen peroxide through at least one dispensing nozzle 2.

In this case, the step of controlling the occurred state transition is a step of controlling the generation of vaporised hydrogen peroxide.

The present invention achieves important advantages.

In the first place, it allows it to be detected for each pulse, therefore for each container, that the state transition to vapour has occurred correctly and consequently that sterilisation has occurred. Furthermore, said check occurs continuously and in proximity to dispensing.

Furthermore, the use of the temperature sensor in the form of filament described allows the outcome of the check to be known in a considerably low response time.

Furthermore, the temperature sensor also facilitates the indirect calculation of the flow rate of atomised liquid as a function of the exposure time with respect to the temperature measured. A possible detection of overdosing (identifiable by means of a temperature that does not return to nominal values even after a period of time from the pulse injection) can send the apparatus into alarm.

## Claims

1. An apparatus (1) for dispensing a flow of a substance, comprising:
at least one dispensing nozzle (2);
a conduit (3) situated upstream of said at least one dispensing nozzle (2) and in direct or indirect fluid communication therewith, a gaseous fluid being intended to flow along said conduit (3);
an atomising nozzle (5) for a liquid, said atomising nozzle (5) being arranged along the conduit (3) to pulse inject the atomised liquid into the conduit (3) to generate said substance;
a control system (6) for an occurred state transition to vapour of the injected atomised liquid, said control system (6) comprising detection means (7) for detecting a temperature variation at or downstream of the atomising nozzle (5).

2. The apparatus (1) according to claim 1, wherein the detection means (7) comprises a temperature sensor active on the conduit (3).

3. The apparatus (1) according to claim 2, wherein the temperature sensor (7) comprises at least one filament of material having conductivity varying with the temperature.

4. The apparatus (1) according to any one of the preceding claims, wherein said control system (6) comprises:
- comparison means (8) for comparing at least a part of the data acquired with a range of temperature values;
- reporting means (9) for reporting an error, adapted to activate when no value acquired within a given time interval following a pulse injection is outside said range of values.

5. The apparatus (1) according to any one of the preceding claims, wherein the detection means (7) is arranged at a first distance from the atomising nozzle (5).

6. The apparatus (1) according to any one of claims 1 to 4, wherein the atomising nozzle (5) comprises the detection means (7).

7. A method for dispensing a flow of a substance, comprising the steps of:
letting a flow of gaseous fluid flow along a conduit (3), said conduit (3) being in direct or indirect fluid communication with at least one dispensing nozzle (2);
during the flowing step, pulse injecting an atomised liquid into the flow of gaseous fluid thus generating said substance;
following at least one pulse injection, controlling an occurred state transition to vapour of the injected atomised liquid, said controlling step comprising a step of detecting a temperature variation in the conduit (3).

8. The method according to claim 7, wherein the liquid is a sterilising agent, said method comprising a step of decontaminating the containers.

9. The method according to claim 8, wherein the liquid is hydrogen peroxide, said method comprising a step of generating vaporised hydrogen peroxide.

10. The method according to any one of claims 7 to 9, comprising a step of comparing at least a part of the data acquired with a range of temperature variation values and a step of reporting an error when no value acquired is outside said range of values.
